# EUROPEAN PATENT SPECIFICATION

(11) **EP 4 429 587 B1**
(45) Date of publication and mention of the grant of the patent: **03.09.2025**
(21) Application number: 22822282.4
(22) Date of filing: 10.11.2022
(51) Int. Cl.: A61C 8/00, A61B 17/16

(54) **DRILL BIT**
BOHRMEISSEL
TRÉPAN

(30) Priority: 13.11.2021 EP 21208105
(43) Date of publication of application: 18.09.2024
(73) Proprietor: Nobel Biocare Services AG, 8302 Kloten (CH)
(72) Inventor: WEITZEL, Jörg, 78239 Rielasingen-Worblingen (DE); BRENNAN, Neill, 8352 Elsau (CH)
(74) Representative: Capré, Didier
(86) International application number: PCT/EP2022/081526
(87) International publication number: WO 2023/083984

(56) References cited:
- WO-A1-2009/066935
- US-A1- 2007 101 827
- US-A1- 2013 274 750
- US-A1- 2019 038 385

## Description

### TECHNICAL FIELD

The present disclosure relates to a drill bit that can be used in dentistry to prepare a recess for receiving a dental implant.

### PRIOR ART

In dentistry, recesses in bone tissues of patients need to be prepared under various circumstances and in particular for the insertion of a dental implant. It is known that proper preparation of an implant-receiving hole has an important influence on the osseointegration of the implant and its long-term success.

Given that the density, orientation and quality of bone differs from patient to patient, it is usually necessary to use several tools to prepare an appropriate implant-receiving recess. However, the more interdependent tools for creating a recess are employed, the more extensive the treatment protocol becomes. Moreover, the alignment of the subsequently used tools can be difficult.

Further, the jawbone has a comparatively hard outer layer, the cortical bone, and underneath a weaker spongy bone structure, the cancellous bone. The cortical bone providing the hard cortex is much denser and less elastic than cancellous bone. Consequently, when preparing a bone recess for receiving a dental implant, a drill bit has to generate this recess extending from the cortical bone into the cancellous bone. In other words, the drill bit is not only confronted with different types of bone between different patients but also within a single patient.

In view of the above, there has been a constant effort to improve the process of creating a recess within bone tissue in preparation for the insertion of an implant. In this respect, WO 2017/129828 A1 discloses a drill bit for preparing a bone recess that is configured to concurrently condense and cut bone tissue.

US2013274750A1 discloses a surgical drill bit with distal cutting and proximal guiding segments, the helical flute has a first leading edge spaced from the central axis by a first length and a second leading edge spaced by a smaller second length.

### SUMMARY OF THE DISCLOSURE

Nonetheless, there remains a demand for a drill bit that enhances the process of creating a recess in bone tissue for the insertion of a dental implant. In particular, it is desirable to enhance the drilling process by taking the different bone layers an implant is to be anchored in into consideration.

In order to address the above, the present disclosure discloses a drill bit that comprises an apical end, a coronal end, a longitudinal axis extending between the apical end and the coronal end, a drill bit core, and a cutting portion extending at least partially along the drill bit core. An outline of a cross-section of the cutting portion perpendicular to the longitudinal axis comprises at least one outermost point at a first radial distance from the longitudinal axis and at least one cutting point at a second radial distance from the longitudinal axis, wherein the cross-section is located along the cutting portion. Further, along a first part of the cutting portion, the second radial distance is smaller than the first radial distance, and along a second part of the cutting portion, the second radial distance is substantially equal to the first radial distance.

The drill bit core may be a substantially cylindrical or conical body. The conical body may have a circular, non-circular, round and/or non-round cross-section, the cross-section being perpendicular to the longitudinal axis of the drill bit.

The outermost point of the cutting portion's cross-section is a point on an outline of the drill bit core's cross-section that has a maximum radial distance from the longitudinal axis. There may be more than one outermost point, i.e. there may be more than one point having the maximum radial distance from the longitudinal axis such as two, three, four, or five outermost points. In other words, there are a number of singular outermost points along the outline of the drill bit core's cross-section.

The cutting portion of the present disclosure relates to a portion of the drill bit, which is configured to cut bone tissue. In this regard, a cutting point is to be understood as a maximum extension of a cutting edge. Generally, the cutting point defines a radial distance (second radial distance) from the longitudinal axis along the outline of the drill bit core's cross-section, where the drill bit core is configured to cut bone tissue. Points along the outline of the drill bit core's cross-section with a radial distance greater than the second radial distance are preferably not configured to cut bone tissue.

To put it differently, the first part of the cutting portion is configured to cut bone tissue along a circle having the second radial distance as a radius. Further, the first part of the cutting portion is not configured to cut bone in a ring-shaped area between the second radial distance and the first radial distance.

In this ring-shaped area, in which the drill bit is not configured to cut bone, the drill bit preferably causes a deformation of the bone tissue while the drill bit rotates in a predetermined direction. In particular, after the cutting point has passed in its direction of rotation, bone tissue that is located radially outwards in relation to the cutting point (i.e. which is located in the ring-shaped area) is compressed radially outwards until the next outermost point is reached.

This compression of bone tissue is advantageous as it increases the bone density and leads to an improved initial stability..

After the outermost point, the outline of the cross-section is configured to allow the radially compressed bone tissue to relax (i.e. to expand radially inwards in relation to the longitudinal axis). Before the next cutting point, the outline particularly extends about the longitudinal axis inside the cutting circle having the radial distance of the cutting point as radius.

Without wishing to be bound by theory, it is assumed that the drill bit takes advantage of the effect that bone tissue having a high density, i.e. hard bone tissue, generally relax or rebounds faster than bone tissue having a low density, i.e. soft bone tissue. As a result, there is a tendency that hard bone tissue is cut more than soft bone tissue. In other words, the drill bit tends to cut bone tissue depending on density of the tissue.

In the second part of the cutting portion, the drill bit has a cutting performance that differs from the cutting performance in the first part of the cutting portion. Since the radial distance of the outermost point and the cutting point are substantially equal, the outline of the second part's cross-section of the cutting portion is generally configured to cut or only cut bone tissue without exerting a compression on the bone tissue. This is basically achieved by having the outermost point and the cutting point that substantially coincide.

As a result, the drill bit has a varying cutting performance along the longitudinal axis, which may be adapted to the bone tissue differing in the depth direction.

According to the invention, the first part of the cutting portion is positioned apically to the second part of the cutting portion.

In this preferred configuration, the above-described cutting performance of the first part of the cutting portion is primarily being applied in a more apical region of the bone in which the bone recess is to be formed, i.e. in particular in cancellous bone tissue. The cutting performance results from the cancellous bone being compressed and cut. Accordingly, the bone tissue is cut to a lesser extent. Without wishing to be bound by theory, it is assumed that the combination of compression and cutting by the drill bit allows for condensing the bone tissue before inserting an implant and due to this enhances primary stability.

Since the second part of the cutting portion is located at a more coronal region of the bone, for example in the region of the cortical bone, and the second part of the cutting portion basically does not condense the bone tissue, a recess of substantially the same shape as the drill bit can be formed that may generally correspond to the shape of the implant to be implanted. Thereby, stress and trauma in this region of the bone can be reduced, which results in less bone resorption. This allows for a faster bone ingrowth of the dental implant, in particular in combination with the primary stability provided in the apical region.

The drill bit may further comprise a non-cutting portion extending along the drill bit core, wherein along the non-cutting portion an outline of a cross-section of the drill bit core perpendicular to the longitudinal axis may comprise at least one outermost point at a first radial distance from the longitudinal axis.

A non-cutting portion represents a portion along the longitudinal axis of the drill bit which is not configured to cut bone tissue. Thereby, this portion is adapted to primarily or only provide a condensing effect on the bone tissue without any intentional cutting action, i.e. without a cutting point being present along the outline of the cross-section.

The non-cutting portion may be positioned apically to the cutting portion. As a result, the non-cutting portion is the first part of the drill bit entering an implantation site for preparing the implantation site for the insertion of an implant. In the recess resulting from the drill bit, the apical end that has been treated by the non-cutting portion provides for a stable primary anchoring of an implant. In case of a non-cutting portion at the apical end, a preparation with a pilot drill before using the drill bit is preferred.

In another aspect of the present disclosure, a drill bit comprises an apical end, a coronal end, a longitudinal axis extending between the apical end and the coronal end, a drill bit core, a cutting portion, and a non-cutting portion. The cutting portion and the non-cutting portion extend along the drill bit core. Along the cutting portion and the non-cutting portion, an outline of a cross-section of the drill bit core perpendicular to the longitudinal axis comprises at least one outermost point at a first radial distance from the longitudinal axis. The outline of the cutting portion further comprises at least one cutting point at a second radial distance from the longitudinal axis. The non-cutting portion is positioned apically to the cutting portion.

The non-cutting portion being located apically of the cutting portion, allows to pre-compress the bone tissue prior to cutting the bone tissue with the cutting portion. This has the advantage that it is possible to exert a condensing effect on a more apical region of the bone tissue, i.e. where cancellous bone is located, without cutting the bone tissue. Thereby, a bone recess for receiving a dental implant is formed that enhances osseointegration of the dental implant.

The cutting portion may include at least a part along the longitudinal axis, where the second radial distance is smaller than the first radial distance.

In other words, the cutting portion may include a part along the longitudinal axis with a ring-shaped area between the second radial distance and the first radial distance as already described above.

The non-cutting portion and/or the cutting portion of the drill bit core along the longitudinal axis may include a compression zone and a relaxation zone.

The compression zone extends along the outline of the drill bit core in a cross-section perpendicular to the longitudinal axis. In the compression zone, the radial distance of the outline from the longitudinal axis increases up to the outermost point when following the outline. Accordingly, a rotation of the drill bit relative to bone tissue (or a point adjacent to the drill bit core) results in the outline of the drill bit pushing the bone tissue (or the point) outwards, i.e. causing the bone tissue to be compressed.

In other words, the compression zone is defined by an increasing radial distance of the drill bit core's outline from the longitudinal axis up to the outline's outermost point in the direction along the outline opposite to the drill bit's predetermined direction of rotation.

The compression zone is a zone along the outline of a cross-section of the drill bit core which is configured, upon rotation of the drill bit, to perform a movement radially outwards towards adjacent bone tissue. This exerts a force on the bone tissue in a direction radially outward resulting in the adjacent bone tissue to be compressed.

After the outermost point of the outline has passed the material (or point), i.e. in the direction opposite to the drill bit's predetermined direction of rotation, the relaxation zone along the outline of the drill bit may begin, where the radial distance of the outline from the longitudinal axis decreases. Accordingly, the rotation of the drill bit relative to the surrounding bone tissue results in a relative radial movement of the outline of the drill bit core inwards (away from the bone tissue). This allows the bone tissue to relax after being compressed by the compression zone.

Thus, the relaxation zone is defined by a decreasing distance of the drill bit core's outline from the longitudinal axis after the outline's outermost point in the direction along the outline and opposite to the drill bit's predetermined direction of rotation.

The relaxation zone is a zone along the outline of a cross-section of the drill bit core which is configured, upon rotation of the drill bit, to perform a movement radially inwards away from adjacent bone tissue. This allows adjacent compressed bone tissue to relax and thereby follow the radial displacement of the drill bit core's outline in a direction radially inwards.

Preferably, the cutting point is positioned within the compression zone.

Positioning the cutting point within the compression zone allows for selectively cutting bone tissue that recovered towards the longitudinal axis of the drill bit after having been compressed in a radial direction away from the longitudinal axis up to the distance of the outermost point. Accordingly, this arrangement of the cutting point allows for an adjustment of the drill bit's cutting behavior to the different areas or types of bone tissue.

The outline of a cross-section along the non-cutting portion of the drill bit core and perpendicular to the longitudinal axis may be non-circular.

Further, the outline of a cross-section along the cutting portion of the drill bit core and perpendicular to the longitudinal axis may be non-circular.

The non-circular outline of the cutting and/or non-cutting portion may be oval, trioval, or quadrioval or more.

A non-circular outline in the present context refers to an outline which differs from an outline of a circle. However, it is preferred that the non-circular outline is substantially rounded, i.e. that the non-circular outline is free from any sharp edges (in case of the cutting portion, except for the part of the outline in relation to the cutting point representing a cutting zone).

Due to the non-circular outline it is possible to form at least one compression zone and at least one relaxation zone and thereby exert a condensing effect on the bone tissue. As noted above, the compression zone is defined along an outline of a cross-section of the drill bit core, in a predetermined direction of rotation, from a maximum radial distance to a minimum radial distance. A relaxation zone is defined along an outline of a cross-section of the drill bit core, in a predetermined direction of rotation, from a minimum radial distance to a maximum radial distance. Depending on the shape of the outline, a plurality of compression zones and relaxation zones may be formed. In particular two, three, four, or five pairs of compression zones and relaxation zones may be formed.

The drill bit core may comprise a guiding portion, the cross-section of the guiding portion perpendicular to the longitudinal axis preferably being circular.

In an embodiment where the drill bit is configured as substantially not having a cutting behavior (cutting point) at the apical portion, preferably a pilot drill is used to form a pilot hole in which the drill bit is insertable. The guiding portion of the drill bit which generally has no cutting or condensing capability facilitates the insertion and guidance of the drill bit and enhances the alignment of the drill bit with the pilot hole.

The cross-section of the drill bit core perpendicular to the longitudinal axis may have two or three pairs of outermost points and cutting points, each pair comprising an outermost point and a cutting point.

Accordingly, a plurality of compression zones and relaxation zones may be formed. Furthermore, a recovery time of the bone tissue may be adjusted by the circumferential positions of the cutting points and outermost points adapting a cutting behavior of the drill bit.

In a predetermined direction of rotation of the drill bit, the location of the cutting point may change from being located before the outermost point to being located at the same location than the outermost point in an apical-coronal direction.

The relative position of the outermost point and the cutting point in relation to the predetermined direction of rotation particularly achieves an adjustment of the type of bone tissue that is at least primarily cut by changing a recovery time of the bone tissue. More specifically, if the cutting point is located after the outermost point in a predetermined direction of rotation of the drill bit (i.e. in an above-mentioned compression zone so that the cutting point passes a location of bone tissue before the outermost point), the bone relaxation time is longer compared with an arrangement where the cutting point is located at the outermost point in a predetermined direction of rotation of the drill bit. Thus, the cutting behavior can be adjusted to different types of bone tissue.

Furthermore, the relative arrangement of the outermost point and the cutting point may change continuously. For example, this allows an adjustment of the drill bit's cutting behavior in accordance with a change in bone structure or density along an insertion path of the drill bit.

The ratio between the first radial distance and the second radial distance between the cross sections of the cutting portion along the longitudinal axis may change, preferably continuously.

The ratio between the first radial distance and the second radial distance in a cross-section of the drill bit core defines the cutting properties of the drill bit core along the longitudinal axis of the drill bit. In other words, a change in the ratio between the first radial distance and the second radial distance along the longitudinal axis results in a change of the cutting behavior of the drill bit.

The cutting portion may comprise at least one cutting flute. It is preferred that the at least one cutting flute extends helically around the drill bit core.

The cutting flute allows an easy formation of the cutting point and is able to collect bone material that has been cut when preparing the bone cavity prior implantation.

The cutting flute is formed as a concavity in the outline of the drill bit in a cross-section perpendicular to the longitudinal axis. The cutting flute forms a cutting zone along the drill bit's outline.

One side of the concavity or cutting zone establishes the cutting point of the drill bit. In particular, the cutting point is formed as a discontinuity along the outline, i.e. a discontinuity at the intersection of the drill bit core's outer surface and the surface of the cutting flute. The cutting point is preferably located at the end of the flute's outline that is following as viewed in the drill bit's predetermined direction of rotation. If the flute is extending helically around the drill bit, the cutting points of the cutting portion's cross-section also extend around the drill bit, in particular with a first pitch.

Preferably, the number of cutting flutes equals the numbers of the outermost points and cutting points. The outermost points of the cross-sections may be located along a helical line having a second pitch, wherein the first pitch and the second pitch differ from each other, the first pitch preferably being smaller than the second pitch.

If the first pitch and the second pitch differ from each other, the relative circumferential positions of the cutting point and the outermost point changes continuously. As described above, the relative positions of the cutting point and the outermost point defines the cutting behavior of the drill bit. Therefore, the difference in the first pitch and the second pitch may continuously adjust the cutting behavior of the drill bit along the cutting portion of the drill bit, preferably corresponding to the change in bone density.

The drill bit may further comprise at least one guiding thread formed helically around the drill bit core and preferably formed as a single thread. Nonetheless, a double or triple thread may also be envisaged.

The guiding thread is configured as a part protruding from the drill bit core that upon a rotation in the predetermined direction pulls the drill bit into the bone tissue or into a pilot hole in the bone tissue. A predetermined speed of the drill bit into the bone tissue is adjustable via the pitch of the guiding thread.

The guiding thread has the advantage that it defines the feed rate of the drill bit into the bone tissue eliminating the dependency on a force exerted by a user. In particular, potentially adverse effects are avoided by the user pushing the drill bit too fast or too slow into the bone of the patient. This may enhance osseointegration of the dental implant.

It is also disclosed a method of preparing an osteotomy, the method comprising: drilling a hole in a jaw bone with a non-round drill having an outline as above described.

### BRIEF DESCRIPTION OF THE DRAWINGS

With the aid of the following figures and description, embodiments are described in more detail for a better understanding of the present disclosure. For this purpose, the features apparent in the figures are marked with reference signs. In this context, essentially the same reference signs are used for different exemplary embodiments, provided that the features of these embodiments are the same or achieve the same effect.
Figure 1 illustrates an exemplary embodiment of a tool for preparing a recess in bone tissue and of cross-sections at different positions along a longitudinal axis of the tool.
Figure 2 illustrates the relationship between a bone recess prepared by a tool according to the present disclosure and an implant to be inserted into this recess.
Figure 3 illustrates an exemplary sequence of tools and an implant to be used for an implantation.

### DETAILED DESCRIPTION OF PREFERRED EMBODIMENTS

In the following, exemplary embodiments of a tool for preparing a recess in bone tissue and, in particular, embodiments of a drill bit according to the present disclosure will be described under reference to the accompanying figures.

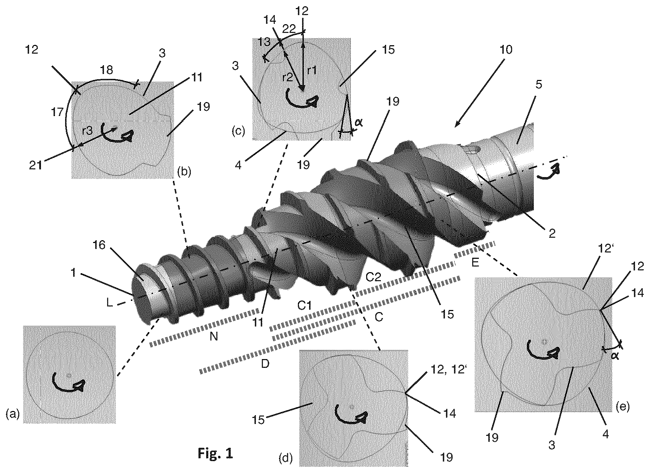

Figure 1 is a perspective view of a drill bit 10 according to the present disclosure. The drill bit 10 comprises an apical end 1, a coronal end 2 and a longitudinal axis L extending between the apical end 1 and the coronal end 2. Further, the drill bit 10 includes a drill bit core 11. The drill bit core 11 preferably extends substantially along the longitudinal axis L between the apical end 1 and the coronal end 2 of the drill bit 10. Adjacent to the coronal end of the drill bit core 11, the drill bit 10 may comprises a coupling portion 5 for coupling the drill bit 10 to another tool (not shown) for applying a predetermined rotation to the drill bit 10.

Figure 1 further illustrates front (a) and cross-sectional views (b) to (e) of the drill bit 10 at different positions along the longitudinal axis L. The cross-sectional views shown in FIG. 1 illustrate cross-sections of the drill bit 10 perpendicular to the longitudinal axis L as viewed in an apical-coronal direction. Each of these front (a) and cross-sectional views (b) to (e) show an outline 3 of the drill bit 10, wherein the outline may include outline sections originating from features such as a cutting flute 15 or a guiding thread 19. Cross-sectional views (c) to (e) additionally include for the sake of comparison each schematically a basic outline 4 of the drill bit core 11, i.e. an outline of the drill bit core 11 representing the basic shape of the drill bit core 11 without any additional features such as a cutting flute 15 or a guiding thread 19. In cross-sectional view (e), the basic outline 3 allows to illustrate where an outermost point 12 of the drill bit core 11 without a cutting flute 15 would be located.

An outline of a cross-section of the drill bit core 11 preferably comprises at least one outermost point 12. The at least one outermost point 12 is substantially located at a first radial distance r1 from the longitudinal axis L. Consequently, the radial distance r1 at the outermost point 12 of the core, represents a maximum radial distance of the outline to the point where the longitudinal axis L intersects the cross-section.

In figure 1, the drill bit core 11 comprises three outermost points 12. However, any other number of outermost points 12 may be formed such as one, two (i.e. oval), three (i.e. trioval), four, five, or six outermost points 12. Preferably, the outermost points 12 are uniformly distributed along the outline of the drill bit core 11.

The cross-sectional views include cross-sections at different portions of the drill bit 10 along the longitudinal axis L. As will be discussed in more detail further below, front view (a) shows a basically circular outline in the non-cutting portion N of the drill bit 10. In other words, the outline does not have an outermost point 12. Cross-sectional view (b) illustrates an outline in a cross-section along the non-cutting portion, wherein the outline includes three outermost points. Cross-sectional view (c) shows a cross-section along the cutting portion C in a condensing portion D of the drill bit 10. Cross-sectional views (d) and (e) illustrate cross-sections coronally of the condensing portion D along the cutting portion C.

Further, in these views (a) to (e), a predetermined direction of rotation of the drill bit 10 is defined in a counter-clockwise direction as indicated by the curved arrows in figure 1.

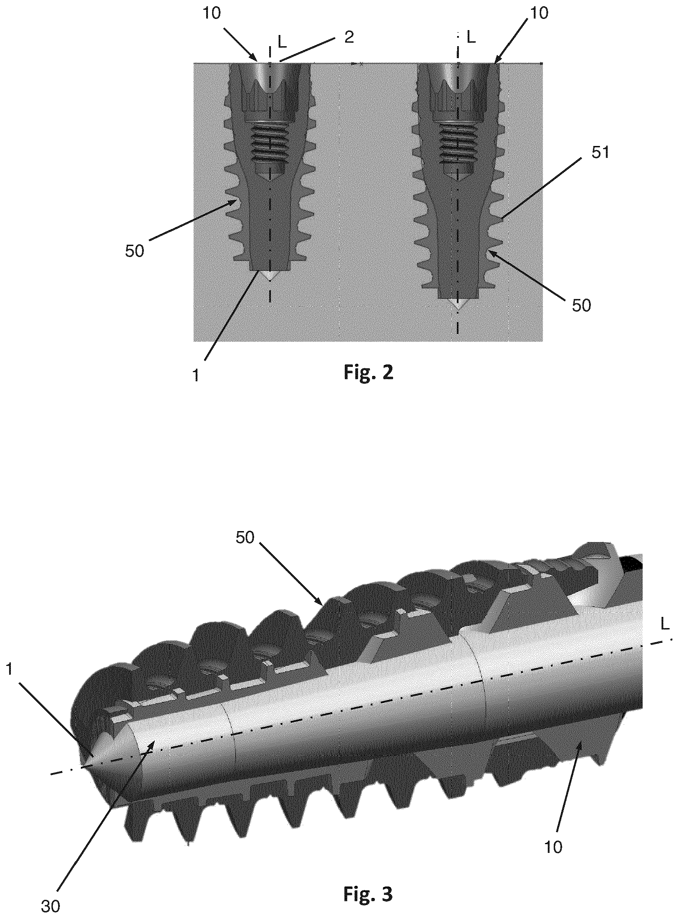

Again turning to the outermost point 12, the drill bit 10 may comprise at least a portion along the longitudinal axis L in an apical-coronal direction, where the diameter and/or the first radial distance r1 of the drill bit core 11 increases. This increase preferably corresponds to an increase in diameter of a dental implant 50 (cf. figure 2) to be implanted into the recess created by means of the drill bit 10.

Further, the drill bit 10 may comprise at least a portion along the longitudinal axis L, where the diameter and/or the first radial distance r1 of the drill bit core 11 remains substantially the same. Such a portion may also be shaped in order to correspond to the implant 50 to be implanted.

At the coronal end 2, the shape of the drill bit 10 may be adapted to have substantially the same size as the dental implant 50 at that location in an implanted state. At the apical end 1, the drill bit 10 may be adapted to be smaller in size than the dental implant 50. The latter is for enhancing primary stability of the implant 50 by causing a press-fit between the patient's bone tissue and the implant 50.

Generally, it is advantageous to adapt the shape of the drill bit 10 along the longitudinal axis L to a geometry of a dental implant 50 to be inserted in a bone recess which is to be prepared with the drill bit 10. Preferably, this adaptation takes the type of bone into consideration that will be located adjacent the implant 50 after implantation. In particular in soft bone, the drill bit 10 is undersized in relation to the implant 50 for supporting the anchorage of the implant 50 within bone tissue.

The same size of the drill bit 10 as the dental implant 50 at the coronal end 2 reduces the strain in the cortical bone tissue after implantation in order to prevent bone resorption and foster bone ingrowth.

The smaller size of the drill bit 10 in relation to the dental implant 50 at the apical end 1 achieves a good first stability of the dental implant 50 in the bone recess.

As already described above, the drill bit core 11 may comprise at least one compression zone 17 and at least one relaxation zone 18 (e.g. cross-sectional view (b) of figure 1).

In the predetermined direction of rotation of the drill bit 10, the at least one compression zone 17 extends along a portion of the outline of a cross-section of the drill bit core 11 starting at the innermost point 21 with the radial distance r3 from the longitudinal axis L preferably to an outermost point 12 along the outline and to a maximum radial distance r1 between the outline and the longitudinal axis L.

Also in the predetermined direction of rotation of the drill bit 10, the at least one relaxation zone 18 extends along a portion of the outline of a cross-section of the drill bit core 11 starting at an outermost point 12 at a maximum radial distance r1 from the longitudinal axis L to an innermost point 21 having the radial distance r3 from the longitudinal axis L.

The outline between an innermost point 21 and an outermost point 12 of the compression zone 17 and/or relaxation zone 18 is preferably smooth, i.e. (only) curved. Nonetheless, either or both zones 17 and 18 may have at least one straight subsection.

It should be noted that an outline of the drill bit core's cross-section having more than one outermost point 12 that are located directly adjacent to each other, i.e. they form a circular line section, is also encompassed since all these outermost points 12 have the first radial distance r1 (maximum radial distance). The same applies to the innermost point 21 at the minimum radial distance r3 from the longitudinal axis L. Nonetheless, having singular outermost point(s) 12 and/or innermost point(s) 21 is also possible.

Preferably, the number of compression zones 17 in the cross-sections of the drill bit core 11 is generally equal to the number of relaxation zones 18. In this context, the term generally is used since either or both zones 17 and 18 may be interrupted by a cutting zone 13 that will be described in more detail below.

In the compression zone 17, the drill bit 10 is configured to compress bone tissue in a radially outward direction, i.e. to exert a densifying or condensing effect on the bone tissue. Upon rotation of the drill bit, a predetermined point in the bone tissue (not shown) is urged radially outward due to the increasing radial distance of the cross-sections of the drill bit core 11 from the radial distance r3 to the radial distance r1. This outward movement relative to the bone tissue causes the bone tissue to be condensed.

In the relaxation zone, the drill bit 10 allows the compressed bone tissue to recover by the outline 3 of the drill bit core 11 moving radially inward. In other words, upon rotation of the drill bit, a predetermined point in the bone tissue (not shown) is able to follow radially inwards due to the decreasing radial distance of the drill bit core's outline from the outermost point 12 to the innermost point 21.

Without wishing to be bound by theory, it has been found that bone tissue with a high density, e.g. cortical bone, generally relaxes or rebounds faster than bone tissue with a low density, e.g. cancellous bone. In other words, in the relaxation zone of the drill bit 10 bone tissue with a higher density relaxes and extends faster radially inward than bone tissue with a lower density.

At least a part of an outline of the cross-section of the drill bit core 11 is preferably non-circular. Accordingly, at least a portion of the drill bit core 11 along the longitudinal axis L has an at least partly non-circular cross-section.

An outline of the cross-section of the drill bit core 11 may also be non-circular along its full extension or at least along substantially the full extension of the drill bit core 11 along the longitudinal axis L. For example, the non-circular outline may be oval or trioval. However, other shapes of non-circular outlines are also possible, in particular a substantially round outline. Nonetheless and as previously discussed, the drill bit core 11 may also comprise an outline of the cross-section at least along a portion of the longitudinal axis L, which is substantially circular.

A non-circular outline allows to define at least one compression zone 17 and relaxation zone 18. That is, the portion of the outline of a cross-section of the drill bit core 11 in a predetermined direction of rotation of the drill bit 10 from a minimum radial distance r3 to a maximum radial distance r1 is defined as a compression zone 17, i.e. it is configured to compress adjacent bone tissue. The portion of the outline of a cross-section of the drill bit core 11 in a predetermined direction of rotation of the drill bit 10 from a maximum radial distance to a minimum radial distance is defined as a relaxation zone 18, i.e. it is configured to allow for relaxation of previously compressed bone tissue. The shape of the non-circular outline 3 may be designed so that a plurality of compression zones 17 and relaxation zones 18 along an outline of the cross-section of the drill bit core 11 are defined.

In some embodiments, the drill bit 10 comprises a guiding thread 19 at least along a portion of the drill bit 10 along the longitudinal axis L. The profile of the guiding thread 19 protrudes radially from the core and extends helically around the drill bit core 11.The guiding thread is preferably not intended to be a screw tap for the implant subsequently placed.

The guiding thread 19 is formed with a predetermined pitch around the drill bit core 11. The guiding thread 19 is preferably formed as a single thread. As noted above, it may also be formed as a double or triple thread.

The guiding thread preferably starts at the apical end. Further, the guiding thread particularly ends apically in relation to the coronal end of the cutting flute 15 described in more detail further below. Coronally of the guiding thread's coronal end, the drill bit 10 preferably includes an elongation portion E. Accordingly, the elongation portion may comprise a cutting flute 15 but preferably does not include a thread. The elongation portion may further be configured to substantially cause no further modification to the recess formed in the adjacent bone tissue. Alternatively, the drill bit 10 may not include a guiding thread 19.

The guiding thread 19 causes the drill bit 10 to be pulled or be guided into the bone tissue with a predetermined speed. The pitch of the guiding thread 19 is preferably adapted so that the drill bit 10 pulls itself into the bone tissue with a predetermined rate (feed per revolution) for creating a satisfying and reproduceable implant receiving hole. As a result, the user does not need to exert a force on the drill bit 10 in order to push the drill bit 10 into the bone tissue. This has the advantage to facilitate the usage of the tool. In other words, the user does not have to worry to feed the tool too fast or too slow into the bone tissue.

In particular if the drill bit 10 is inserted into a pilot hole (not shown) that has been previously prepared, the guiding thread 19 also assists in an alignment of the drill bit 10 with the hole. Thereby, a misalignment of the tool by a user is more effectively prevented. This contributes to achieving a desired shape of the bone recess and may foster the osseointegration of the implant and its long-term success.

Along the longitudinal axis L, the drill bit 10 comprises a cutting portion C. The drill bit 10 may further include a condensing portion D, a non-cutting portion N, and/or an elongation portion E along the longitudinal axis L and a guiding area 16. The condensing portion D may partially or fully overlap with the cutting portion C and/or the non-cutting portion N.

Including some or all of above-noted portions along the longitudinal axis of the drill bit 10 allows for an adaption of the drill bit 10 to different densities of the bone tissue. In particular, the drill bit 10 is configured differently at the coronal end 2, where the drill bit 10 is preparing cortical bone tissue with a compact structure and a comparatively high density, than at the apical end, where the drill bit 10 is generally preparing cancellous bone tissue with a trabecular structure and a generally lower density. It is thus possible to prepare these different regions of bone tissue in a single step when preparing the bone recess for inserting a dental implant. In this way, complex drill protocols may be avoided in most patients. Nonetheless, a pilot drill 30 may be employed prior the drill bit 10 to facilitate preparation.

All portions along the longitudinal axis of the drill bit 10 are described in the following in more detail with reference to Figure 1.

The drill bit 10 comprises at least one cutting portion C. The cutting portion C preferably extends at least partially along the drill bit core 11 along the longitudinal axis L. Preferably, the cutting portion C does not extend to the apical end 1 of the drill bit 10 or drill bit core 11. In other words, the drill bit 10 may not be configured for cutting bone tissue at the apical end 1. Alternatively, the cutting portion C may be present along substantially the full length of the drill bit core 11 along the longitudinal axis L (i.e. without the coupling portion 5).

In any case, the apical end may be used to guide the drill bit 10 into the bone tissue of a patient.

The cutting portion C may at least partly overlap with the condensing portion D. That is, the cutting portion C may also at least partly be configured as condensing portion D of the drill bit 10 and vice versa.

As illustrated in figure 1, the cutting portion C is tapering apically, i.e. the cross-sections of the cutting portion C perpendicular to the longitudinal axis L decrease in size from the coronal end of a cutting portion C to an apical end of a cutting portion C. As a result, when the drill bit drills into the bone tissue, the cutting portion increases the size of the hole that is created by cutting adjacent bone tissue. If a guiding thread 19 is present, the cutting motion is performed with a particular steady feed and in a particular helical manner.

If the cutting portion C is at least partly or entirely also configured as a condensing portion D, a cross-section of this cutting portion C preferably comprises a compression zone 17 and a relaxation zone 18. Thereby, such a cutting portion C is configured to compress bone tissue in the compression zone 17 and to allow the bone tissue to relax in the relaxation zone 18 while rotating in the predetermined direction of the drill bit 10.

The profile along and/or the transition between the compression zone 17 and relaxation zone 18 is preferably (only) curved, i.e. it may not include a straight section or a discontinuity (except for a cutting zone 13 in case of a cutting portion C as will be described in more detail below). This has a positive effect on the structural integrity of the bone tissue that is treated with the drill bit 10.

It should be noted that in the present disclosure a condensing portion D, if present and having a compression zone 17 and a relaxation zone 18, includes both a compression zone 17 and a relaxation zone 18 (i.e. not only a compression zone 17 and not only a relaxation zone 18).

Nonetheless, at least a part of or the entire cutting portion C may also be configured as a cutting portion C without being configured as a condensing portion D. Consequently, such a cutting portion is configured without compression and relaxation zones.

Preferably, the outline of a cross-section of the drill bit core 11 along at least a part of or the entire cutting portion C is non-circular. Even more preferably, the outline is non-circular and includes a compression zone 17 and a relaxation zone 18. This results in the outline being configured as a condensing portion D. Thereby, the drill bit 10 can exert a condensing effect on the bone tissue. Depending on the configuration of the non-circular outline, a plurality of compression zones and relaxation zones along an outline of the cross-section of the drill bit core 11 can be defined.

If not being configured as a condensing portion D and in particular if also not being configured as a cutting portion C, the outline of the cross-section of the drill bit core 11 may be substantially circular. The outline of a cross-section of a cutting portion C that is not configured as a condensing portion D is to be understood as being substantially circular if a cutting flute 15 and/or a guiding thread 19 is present.

Along an outline of a cutting portion's cross-section of the drill bit core 11, the cutting portion C preferably comprises at least one cutting zone 13 including a cutting point 14. The cutting zone 13 may be defined by a cutting flute 15, which forms a concave recess in the outline of the cutting portion C. Accordingly, the cutting portion C may further comprise at least one cutting flute 15. The cutting flute 15 is preferably formed as a recess or groove in and extending along the drill bit core 11. Further, the cutting flute 15 may be straight but preferably extends helically around the drill bit core 11. The latter case distributes the cutting force along the circumference of the drill bit 10 and facilitates guidance during insertion.

Preferably, the cutting zone 13 including the cutting point 14 is located in the compression zone 17 of the outline 4 of a cutting portion C that is also configured as a condensing portion D. In this case, the outline of the compression zone 17 is interrupted by the cutting zone 13.

The at least one cutting point 14 is located at a second radial distance r2 from the longitudinal axis L in a cross-sectional view of the drill bit core 11 along the cutting portion C. An outline 4 of the cross-sections of the drill bit core 11 along the cutting portion C may comprise two or three pairs of outermost points 12 and cutting points 14, wherein each pair comprises an outermost point 12 and a cutting point 14.

A cutting point 14 preferably represents a discontinuity along the outline 4 of a cutting portion C. Accordingly, the cutting point comprises a clearance angle, a cutting angle, and a rake angle.

The at least one cutting point 14 located at a second radial distance r2 allows to cut bone tissue along a circle within the cross-section having the second radial distance r2 from the longitudinal axis L as a radius. If the second radial distance r2 is smaller than the a first radial distance r1 of an outermost point 12 described in more detail below, the drill bit 10 is not configured to cut bone tissue in an area having a radial distance that is larger than the second radial distance r2.

Thus, the second radial distance r2 may be smaller than the first radial distance r1 of the outermost point 12 in a cross-section of the drill bit core 11 in at least a part of the cutting portion C along the longitudinal axis L, i.e. at least in some of the cross-sections along the longitudinal axis L. In other words, the drill bit 10 and the cutting portion C may comprise a portion along the longitudinal axis L with a negative clearance angle α.

Alternatively, the first radial distance r1 and the second radial distance r2 may also be substantially equal in a cross-section of the drill bit core 11 in at least a portion of the cutting portion C along the longitudinal axis L. In other words, the outermost point 12 and the cutting point 14 may substantially coincide. In this case, the drill bit 10 and the cutting portion C may comprise a portion along the longitudinal axis, where the cutting point 14 has a positive clearance angle α.

Preferably, the ratio between the first radial distance r1 of the outermost point 12 and the second radial distance r2 of the cutting point 14 changes from cross-section to cross-section of the drill bit core 11 along the cutting portion C.

In an embodiment, where the second radial distance r2 is smaller than the first radial distance r1, the drill bit 10 and the cutting portion C comprise a non-cutting zone 22 where the drill bit 10 is not configured to cut bone (cf. cross-section (c) of figure 1). The non-cutting zone 22 is preferably part of the compression zone 17.

The radial extension of the non-cutting zone 22 defines a ring-shaped area between the second radial distance r2 and the first radial distance r1. Within this non-cutting zone 22, the drill bit 10 is configured to alternately compress or allow for relaxation of bone tissue, i.e. to exert a force on the bone tissue in the radial direction without cutting the bone tissue. As described above, in the compression zone 17, bone tissue adjacent to the compression zone is urged radially outward upon rotation of the drill bit 10 in the predetermined direction of rotation, whereas in the relaxation zone 18, a recovery of bone tissue in a direction radially inward is allowed.

Without wishing to be bound by theory, the drill bit 10 takes advantage of the observation that bone tissue with a greater density relaxes faster, i.e. moves faster radially inward, than bone tissue with a lower density. Subsequently to compressing bone tissue in the compression zone 17 in a direction radially outward, the compressed bone tissue recovers (i.e. moves) radially inward in the relaxation zone 18 . Due to the difference in recovery time, the drill bit 10 has a bias toward cutting bone tissue with a greater density (e.g. cortical bone tissue).

The time allowed for recovery is dependent on the circumferential position of the cutting point 14 and the outermost point 12 (and the rotation rate or speed of the drill bit 10) and, thus, the location of the compression zone 17 and the relaxation zone 18 along the outline 4 of a cross-section of the drill bit core 11 in relation to the cutting point 14. Bone tissue, which recovers in the predetermined allowed recovery time to a radial distance smaller than the second radial distance r2 is to be cut at the next cutting point 14 passing by, whereas bone tissue which recovers to a point between the second radial distance r2 and the first radial distance r1 is not to be cut at the next cutting point 14. The cutting point 14 is thus able to cut bone tissue to a different degree, i.e. to cut hard bone tissue to a higher degree than soft bone tissue. This effect can be adjusted via the geometry of the drill bit core 10, i.e. the magnitude of the first and second radial distances r1 and r2 as well as the circumferential positions thereof.

In a case, in which the second radial distance r2 is substantially equal to the first radial distance r1, the drill bit 10 and the cutting portion C have a cutting behaviour that is different from the previously described cutting behavior. Since the cutting point 14 and the outermost point 12 substantially coincide, the cutting point 14 is located at the most radial outward position. In other words, all other points on an outline of the cross-section of the drill bit core 11 are located more radially inward than the cutting point 14. Therefore, the drill bit 10 is not configured to compress or allow for relaxation of bone tissue but, instead, cuts bone tissue in a circular area spanned by the first radial distance r1 or the second radial distance r2.

This is structurally illustrated in cross-sections (d) and (e) of figure 1. In cross-section (d), the theoretical outermost point 12' of the basic outline 4 of the drill bit core 11 (i.e. the outline not considering a guiding thread 19 and/or a cutting flute 15) as well as the outermost point 12 of the drill bit's outline 3 coincide with the cutting point 14 of the drill bit's outline 3.

It should be noted that the determination of the outermost point 12 of the drill bit's outline 3 does not take a guiding thread 19 into account, if present (cf. cross-sections (c) to (e) of figure 1). Further, albeit cross-sections perpendicular to the longitudinal axis along the cutting portion C may generally comprise a predetermined number of compression zones 17, relaxation zones 18, cutting zones 13, non-cutting zones 22, outermost points 12, and/or cutting points 14, certain cross-sections may not include all of these features due to an influence of other structural features of the drill bit 10, in particular the guiding thread 19 (cf. cross-section (c) of figure 1 missing one relaxation zone and part of one compression zone due to the guiding thread 19).

Accordingly, the outline is not configured to include a compression zone 17 and relaxation zone 18. Along the part of the outline, where a compression zone 17 would be located is, instead, a recess formed by a cutting flute 15. In other words, instead of a compression zone 17 a cutting zone 13 is arranged along the outline of the drill bit 10.

Turning to cross-section (e) of figure 1, the theoretical outermost point 12' of the basic outline 4 of the drill bit core 11 is located along the cutting zone 13, where the cutting flute 15 along the outline 3 of the drill bit 10 is formed. Thus, the theoretical outermost point 12' does not coincide with the outermost point 12 of the drill bit's outline 3. However, also in this case the outermost point 12 coincides with the cutting point 14. Likewise, cross-section (e) does not include a compression zone 17 or a relaxation zone 18. Although a part of the outline corresponds structurally to a relaxation zone 18, it fails to do this in terms of its functional configuration. In other words, the outline 3 lacks a compression zone 17 having the function to compress bone tissue so that there cannot be a relaxation zone 18 that allows for a relaxation of bone tissue that has previously been compressed by a compression zone 17 of the drill bit 10.

The skilled person will appreciate from the explanation above that the cutting behavior of the drill bit 10 changes with a change in the ratio between the first radial distance r1 and the second radial distance r2. Accordingly, it is possible to adjust the cutting behavior of the drill bit 10 along the longitudinal axis L, in particular taking different regions in the depth direction of bone tissue into account.

Preferably, the cutting portion C comprises a first part C1 along the longitudinal axis L with the second radial distance r2 of the cutting point 14 being smaller than the first radial distance r1 of the outermost point 12. In this first part C1, the cutting portion C may comprise a negative clearance angle α at the cutting point 14. Further, the first part C1 of the cutting portion C may also be configured as a condensing portion D. Accordingly, an outline of a cross-section of the first part C1 of the cutting portion C preferably comprises at least one compression zone 17 and at least one relaxation zone 18.

The cutting portion C may further comprise a second part C2. In the second part C2 of the cutting portion C, a second radial distance r2 may substantially be equal to the first radial distance r1. The second part C2 of the cutting portion C may further comprise a cutting point 14 with a positive clearance angle α. The second part C2 of the cutting portion C may not form a condensing portion D of the drill bit 10. Accordingly, the second part C2 of the cutting portion C does preferably not comprise a compression zone 17 or a relaxation zone 18. The first part C1 of the cutting portion C is positioned apically to the second part C2 of the cutting portion.

Due to the difference in ratio of the first radial distance r1 and the second radial distance r2 between the first part C1 and the second part C2 of the cutting portion C, the cutting behavior of these parts differ from each other.

In particular, the outline of the first part C1 of the cutting portion C is configured, upon rotation of the drill bit, to compress bone tissue in the compression zone 17, allow for relaxation of the bone tissue in the relaxation zone 18, and then cut the bone tissue at the cutting point 14 of the cutting zone 13. As a result, the amount of bone tissue being cut upon rotation depends on the relaxation properties of the bone tissue, i.e. how much and how fast does the bone tissue relax after being compressed.

Without wishing to be bound by theory, the inventors observed that cancellous bone with a relatively low bone density is cut less. Accordingly, more of the soft bone tissue remains for supporting the dental implant to be inserted.

The second part C2 of the cutting portion C is not configured to compress bone tissue upon rotation but, instead, cuts bone tissue at the outermost point 12 corresponding to the cutting point 14.

Without wishing to be bound by theory, this feature is advantageous in the cortical bone with a relatively high bone density where further condensing of bone tissue has less effect. Accordingly, the second part preferably creates a bone recess in the cortical region of the bone tissue with a size substantially corresponding to the size of the dental implant 50 for avoiding bone resorption and, thus, a faster ingrowth of the dental implant 50.

In the predetermined direction of rotation of the drill bit 10, the cutting point 14 along the first part C1 may be located along the outline of the drill bit's cross-section so that upon rotation the cutting point 14 passes a bone tissue location before or at the same time as the outermost point 12. After the relative position of the outermost point 12 and the cutting point 14 has changed along the outline so that they correspond to each other, the theoretical outermost point 12' may continue to change its relative position to pass a bone tissue location before the cutting point. This causes a continuous change in the clearance angle α from a negative to a positive clearance angle.

As explained above, the geometry of the drill bit 10 and, in particular, the magnitude of the first and second radial distances r1 and r2 as well as the circumferential positions thereof define the cutting behavior of the drill bit 10.

For a change of the cutting behaviour, the outermost points 12 of the cross-sections of the drill bit core 11 along the longitudinal axis L may be located along a helical line with a second pitch around the longitudinal axis L. Alternatively, the outermost points 12 of these cross-sections may also be located along a substantially straight line that is preferably arranged parallel to the longitudinal axis L.

As mentioned above, the cutting flute may extend helically around the drill bit 10. Accordingly, the cutting points 14 also extend helically around the drill bit 10, with a first pitch. The first pitch may be the same pitch as the second pitch of the outermost points 12 or may be different than the second pitch of the outermost points 12. In particular, the first pitch may be smaller than the second pitch.

Preferably, the first pitch of the cutting points 14 and the second pitch of the outermost points 12 differ from each other. In particular, the first pitch is smaller than the second pitch. Alternatively, the first and second pitch may substantially be equal.

In an embodiment where the first pitch and the second pitch differ from each other, the relative circumferential position of the outermost point 12 and the cutting point 14 changes along the longitudinal axis L of the cutting portion C of the drill bit 10. It is thus possible to continuously adjust the cutting behavior of the drill bit 10 along the longitudinal axis L to different regions of the bone.

The drill bit 10 may further comprise a non-cutting portion N extending along the longitudinal axis L of the drill bit 10. In the non-cutting portion N, the drill bit 10 does not comprise a cutting point 14 or a cutting zone 13. In other words, the drill bit 10 is not configured to cut bone tissue in this non-cutting portion N.

Preferably, the non-cutting portion N is located apically to the cutting portion C.

The non-cutting portion N may at least partly overlap with a condensing portion D along the drill bit 10. Thus, the non-cutting portion N may at least partly be also configured as a condensing portion D of the drill bit 10. Accordingly, the outline of a cross-section of the drill bit core 11 along the non-cutting portion N may include at least one compression zone 17 and at least one relaxation zone 18.

Thereby, it is possible to exert a condensing effect on the bone tissue along the non-cutting portion. Depending on the shape of the non-circular outline and as already described above, the outline may further be configured to comprise a plurality of compression zones 17 and relaxation zones 18.

If the non-cutting portion N is located apically of the cutting portion C, it is possible to exert a condensing effect on the bone tissue prior cutting the bone tissue. Thereby, the bone tissue may already be compressed before the bone tissue is first cut. This enhances the guidance of the drill bit 10 into the bone tissue due to the absence of cutting forces on the one hand and pre-condenses the bone tissue.

Accordingly, the outline of the cross-sections of the drill bit core 11 along at least a portion of or the entire non-cutting portion N along the longitudinal axis L may be non-circular.

The non-cutting portion N may also comprise cross-sections along at least a portion of or its entirety having a substantially circular outline. The non-cutting portion N may further comprise a substantially circular guiding portion 16. In this case, an outline of a cross-section of the drill bit core 11 along the guiding portion 16 is substantially circular.

Preferably, the guiding portion 16 is located apically to the cutting portion C and, more preferably, apically to a portion of the non-cutting portion N that comprises a non-circular outline in a cross-section of the drill bit core 11 perpendicular to the longitudinal axis L. In other words, the guiding portion 16 may be the most apical portion of the drill bit 10.

The guiding portion 16 may also be formed as or comprise a conical guiding tip (not shown) at the apical end of the drill bit 10. Alternatively, the drill bit 10 may not comprise a guiding portion 16.

In a case, in which the drill bit 10 has an apical portion that is not configured to cut bone tissue, preferably a pilot hole using a pilot drill is drilled prior to preparing the bone recess using the drill bit 10. In particular here, the guiding portion 16 enhances an alignment of the drill bit 10 and a pilot hole and facilitates insertion of the drill bit 10.

As already described above, the drill bit 10 may comprise a condensing portion D extending along a portion of the drill bit core 11. The condensing portion D may at least partly overlap with the non-cutting portion N and/or the cutting portion C. In a preferred embodiment, the condensing portion D overlaps at least partly with a first part C1 of the cutting portion C. Even more preferably, the condensing portion D overlaps at least partly with a non-cutting portion N that comprises a non-circular outline.

The condensing portion D is configured to cause a compression and relaxation cycle of bone tissue upon rotation of the drill bit 10, which is used to prepare the bone recess for implantation.

The condensing portion D may not be included in an apical portion of the drill bit 10, i.e. the condensing portion D may not be present in the guiding portion 16 of the non-cutting portion N and/or in a coronal portion of the drill bit core 11. Preferably, the condensing portion D is not present along the second part C2 of the cutting portion C. Alternatively, the condensing portion D may extend along the entire drill bit core 11.

As previously discussed, an outline of the cross-section of the drill bit core 11 along the condensing portion D preferably comprises at least one compression zone 17 and at least one relaxation zone 18. In the condensing portion D, an outline of the cross-section of the drill bit core 11 may be non-circular. Further, a cutting point 14 may be present in at least a part of the condensing portion D or not. If at least a part of the condensing portion D comprises a cutting point 14, a first radial distance r1 of the outermost point 12 is preferably larger than a second radial distance r2 of the cutting point 14.

Figure 2 illustrates a relationship between a bone recess prepared by a drill bit 10 according to the present disclosure and a dental implant 50 to be inserted into the recess for two different lengths of the dental implant 50. The dental implant 50 comprises an external thread 51 that anchor the dental implant within the surrounding bone tissue.

As illustrated in Figure 2, the drill bit 10 preferably has at its coronal end portion (i. e. in the cortical region of the bone tissue) substantially the same size as the dental implant 50. In other words, the coronal end portion at the coronal end 2 of the drill bit 10 may have substantially the same size as the outer diameter of the corresponding portion of the dental implant 50.

These dimensions of the drill bit 10 relative to the dental implant allows for a sufficient seal at the opening of the recess prepared in the bone tissue and is configured to to minimize, or to reduce the strain in the cortical bone portion causing bone resorption. This has a positive effect on healing time and serves as a basis for enhancing bone ingrowth into the dental implant 50 in this region.

As Figure 2 also illustrates, the drill bit 10 may decrease in size relative to the dental implant 50. More specifically, the relative size of the drill bit's cross sections along the longitudinal axis in a coronal-apical direction becomes smaller. That is, apically the drill bit 10 may comprise a maximum extension perpendicular to the longitudinal axis that becomes less than 30%, less than 40%, less than 50%, less than 60% or less than 70% of the outer diameter of the dental implant 50. Preferably, this relationship of the size between the drill bit 10 and the dental implant 50 extends along 40%, 50% or 60% of the length of the drill bit 10 and/or the dental implant 50 starting from the apical end 1 towards the coronal end 2.

The greater outer diameter of the dental implant 50 in the cancellous bone portion relative to the size of the drill bit 10 results in an enhanced initial stability of the dental implant 50 in the bone recess. This stability fosters the anchorage of the dental implant 50 by providing the basis for bone ingrowth.

Figure 3 is a perspective view illustrating the relationship between a pilot drill 30, the drill bit 10, and the dental implant 50. More specifically, Figure 3 illustrates the overlap of the pilot drill 30, the drill bit 10 and the dental implant 50 when being inserted into bone tissue and, thus, an exemplary procedure for preparing a bone recess for the dental implant 50.

A pilot drill 30 may be used in a first step for drilling a pilot hole into the bone that has a diameter that is generally smaller than the corresponding size of drill bit 10. However, the guiding portion 16 of drill bit 10 may have a diameter that is equal or smaller than the diameter of the pilot drill. The pilot hole created by the pilot drill 30 serves as a guiding hole for the subsequent steps.

Since the exemplary drill bit 10 illustrated in figure 3 comprises a non-cutting portion N at an apical end 1 thereof the drill bit 10 or, the guiding portion 16 in combination with the guiding thread 19 in the non-cutting portion N of the drill bit 10 guides the drill bit into the previously drilled pilot hole.

Then, the drill bit 10 may be used to enlarge the pilot hole created by the pilot drill 30 for preparing the bone recess for insertion of the dental implant 50. Accordingly, the drill bit 10 is inserted into the pilot hole and the guiding thread 19 of the drill bit 10 preferably pulls the drill bit 10 into the pilot hole with a predetermined rate corresponding to the rotation of the drill bit 10. If present, a guiding thread has the advantage to eliminate the need for the user to exert a force on the drill bit 10 in order to move the drill bit 10 into the pilot hole.

After preparation of the bone recess, the dental implant 50 is inserted.

With the drill bit 10 according to the present disclosure, it is possible to reduce the drill protocol to a minimum of three steps. A sequential use of a plurality of different tools and complex drill protocols may thus be avoided and a misalignment of sequentially used tools may be more easily prevented.

### REFERENCE SIGNS

- 1: apical end
- 2: coronal end
- 3: outline of the drill bit
- 4: basic outline of the drill bit core
- 5: coupling portion
- 10: drill bit
- 11: drill bit core
- 12: outermost point
- 12': theoretical outermost point
- 13: cutting zone
- 14: cutting point
- 15: cutting flute
- 16: guiding portion
- 17: compression zone
- 18: relaxation zone
- 19: guiding thread
- 21: innermost point
- 22: non-cutting zone
- 30: pilot drill
- 50: dental implant
- 51: external thread

- C: cutting portion
- C1: first part of the cutting portion
- C2: second part of the cutting portion
- D: condensing portion
- E: elongation portion
- L: longitudinal axis
- N: non-cutting portion
- r1: first radial distance
- r2: second radial distance
- r3: minimum radial distance

- α: clearance angle

## Claims

1. A drill bit (10) comprising:
an apical end (1), a coronal end (2), a longitudinal axis (L) extending between the apical end and the coronal end,
a drill bit core (11), and
a cutting portion (C) extending at least partially along the drill bit core;
wherein an outline of a cross-section of the cutting portion perpendicular to the longitudinal axis comprises at least one outermost point (12) at a first radial distance (r1) from the longitudinal axis and at least one cutting point (14) at a second radial distance (r2) from the longitudinal axis; and
wherein along a first part of the cutting portion, the second radial distance is smaller than the first radial distance, and along a second part of the cutting portion, the second radial distance is substantially equal to the first radial distance.
and wherein the first part of the cutting portion (C) is positioned apically to the second part of the cutting portion.

2. The drill bit (10) according to claim 1, wherein the drill bit further comprises a non-cutting portion (N) extending along the drill bit core (11), wherein along the non-cutting portion an outline of a cross-section of the drill bit core perpendicular to the longitudinal axis (L) comprises at least one outermost point (12) at a first radial distance (r1) from the longitudinal axis.

3. The drill bit (10) according to claim 2, wherein the non-cutting portion (N) is positioned apically to the cutting portion (C).

4. The drill bit (10) according to claim 1 further comprising:
a non-cutting portion (N)extending along the drill bit core;
wherein along the non-cutting portion an outline of a cross-section of the drill bit core perpendicular to the longitudinal axis comprises at least one outermost point (12) at a first radial distance (r1) from the longitudinal axis,
wherein the non-cutting portion is positioned apically to the cutting portion.

5. The drill bit (10) according to claim 4, wherein the cutting portion (C) includes at least a part along the longitudinal axis, where the second radial distance (r2) is smaller than the first radial distance (r1).

6. The drill bit (10) according to claim 4 or 5, wherein the non-cutting portion (N) of the drill bit core (11) includes a compression zone and a relaxation zone.

7. The drill bit (10) according to any one of claims 4 to 6, wherein the outline of a cross-section along the non-cutting portion (N) of the drill bit core (11) and perpendicular to the longitudinal axis (L) is non-circular.

8. The drill bit (10) according to any one of the preceding claims, wherein the cutting portion (C) of the drill bit core (11) includes a compression zone (17) and a relaxation zone (18).

9. The drill bit (10) according to claim 8, wherein the cutting point (14) is positioned within the compression zone.

10. The drill bit (10) according to any one of the preceding claims, wherein the outline of a cross-section along the cutting portion (C) of the drill bit core (11) and perpendicular to the longitudinal axis (L) is non-circular.

11. The drill bit (10) according to any one of the preceding claims, wherein extending from the apical end (1) towards the coronal end (2), the drill bit core further comprises a guiding portion (16), the cross-section of the guiding portion perpendicular to the longitudinal axis (L) preferably being circular.

12. The drill bit (10) according to any one of the preceding claims, wherein the cross-section of the drill bit core (11) perpendicular to the longitudinal axis (L) has two or three pairs of outermost points (12) and cutting points (14), each pair comprising an outermost point and a cutting point.

13. The drill bit (10) according to any one of the preceding claims, wherein in a predetermined direction of rotation of the drill bit, the location of the cutting point (14) changes from being located before the outermost point (12) to being located after the outermost point in a apical-coronal direction.

14. The drill bit (10) according to any one of the preceding claims, wherein the ratio between the first radial distance (r1) and the second radial distance (r2) between the cross sections of the cutting portion (C) along the longitudinal axis (L) changes.

15. The drill bit (10) according to any one of the preceding claims, wherein the cutting portion (C) comprises at least one cutting flute (15).

16. The drill bit (10) according to claim 15, wherein the at least one cutting flute (15) extends helically around the drill bit core (11) with a first pitch.

17. The drill bit (10) according to claim 16, wherein along the longitudinal axis (L), the outermost points (12) of the cross-sections are located along a helical line having a second pitch, wherein the first pitch and the second pitch differ from each other, the first pitch preferably being smaller than the second pitch.

18. The drill bit (10) according to any one of the preceding claims, wherein the drill bit further comprises at least one guiding thread (19) formed helically around the drill bit core (11) and preferably formed as a single thread.

## Patentansprüche

1. Ein Bohrer (10), umfassend:
ein apikales Ende (1), ein koronales Ende (2), eine Längsachse (L), die sich zwischen dem apikalen Ende und dem koronalen Ende erstreckt,
einen Bohrkern (11) und
einen Schneidabschnitt (C), der sich zumindest teilweise entlang des Bohrkerns erstreckt;
wobei ein Umriss eines Querschnitts des Schneidabschnitts senkrecht zur Längsachse mindestens einen äussersten Punkt (12) in einem ersten radialen Abstand (r1) von der Längsachse und mindestens einen Schneidpunkt (14) in einem zweiten radialen Abstand (r2) von der Längsachse umfasst; und
wobei entlang eines ersten Teils des Schneidabschnitts der zweite radiale Abstand kleiner ist als der erste radiale Abstand, und entlang eines zweiten Teils des Schneidabschnitts der zweite radiale Abstand im Wesentlichen gleich dem ersten radialen Abstand ist;
und wobei der erste Teil des Schneidabschnitts (C) apikal zum zweiten Teil des Schneidabschnitts positioniert ist.

2. Der Bohrer (10) nach Anspruch 1, wobei der Bohrer ferner einen nicht schneidenden Abschnitt (N) umfasst, der sich entlang des Bohrkerns (11) erstreckt, wobei entlang des nicht schneidenden Abschnitts ein Umriss eines Querschnitts des Bohrkerns senkrecht zur Längsachse (L) mindestens einen äussersten Punkt (12) in einem ersten radialen Abstand (r1) von der Längsachse umfasst.

3. Der Bohrer (10) nach Anspruch 2, wobei der nicht schneidende Abschnitt (N) apikal zum Schneidabschnitt (C) positioniert ist.

4. Der Bohrer (10) nach Anspruch 1, ferner umfassend:
einen nicht schneidenden Abschnitt (N), der sich entlang des Bohrkerns erstreckt;
wobei entlang des nicht schneidenden Abschnitts ein Umriss eines Querschnitts des Bohrkerns senkrecht zur Längsachse mindestens einen äussersten Punkt (12) in einem ersten radialen Abstand (r1) von der Längsachse umfasst,
wobei der nicht schneidende Abschnitt apikal zum Schneidabschnitt positioniert ist.

5. Der Bohrer (10) nach Anspruch 4, wobei der Schneidabschnitt (C) mindestens einen Teil entlang der Längsachse umfasst, wo der zweite radiale Abstand (r2) kleiner ist als der erste radiale Abstand (r1).

6. Der Bohrer (10) nach Anspruch 4 oder 5, wobei der nicht schneidende Abschnitt (N) des Bohrkerns (11) eine Kompressionszone und eine Relaxationszone umfasst.

7. Der Bohrer (10) nach einem der Ansprüche 4 bis 6, wobei der Umriss eines Querschnitts entlang des nicht schneidenden Abschnitts (N) des Bohrkerns (11) und senkrecht zur Längsachse (L) nicht kreisförmig ist.

8. Der Bohrer (10) nach einem der vorhergehenden Ansprüche, wobei der Schneidabschnitt (C) des Bohrkerns (11) eine Kompressionszone (17) und eine Relaxationszone (18) umfasst.

9. Der Bohrer (10) nach Anspruch 8, wobei der Schneidpunkt (14) innerhalb der Kompressionszone positioniert ist.

10. Der Bohrer (10) nach einem der vorhergehenden Ansprüche, wobei der Umriss eines Querschnitts entlang des Schneidabschnitts (C) des Bohrkerns (11) und senkrecht zur Längsachse (L) nicht kreisförmig ist.

11. Der Bohrer (10) nach einem der vorhergehenden Ansprüche, wobei sich von dem apikalen Ende (1) in Richtung des koronalen Endes (2) erstreckend der Bohrkern ferner einen Führungsabschnitt (16) umfasst, wobei der Querschnitt des Führungsabschnitts senkrecht zu der Längsachse (L) vorzugsweise kreisförmig ist.

12. Der Bohrer (10) nach einem der vorhergehenden Ansprüche, wobei der Querschnitt des Bohrkerns (11) zwei oder drei Paare von äussersten Punkten (12) und Schneidpunkten (14) senkrecht zu der Längsachse (L) aufweist, wobei jedes Paar einen äussersten Punkt und einen Schneidpunkt umfasst.

13. Der Bohrer (10) nach einem der vorhergehenden Ansprüche, wobei sich in einer vorbestimmten Drehrichtung des Bohrers die Lage des Schneidpunkts (14) von einer Lage vor dem äussersten Punkt (12) zu einer Lage nach dem äussersten Punkt (12) in einer apikal-koronalen Richtung ändert.

14. Der Bohrer (10) nach einem der vorhergehenden Ansprüche, wobei sich das Verhältnis zwischen dem ersten radialen Abstand (r1) und dem zweiten radialen Abstand (r2) zwischen den Querschnitten des Schneidabschnitts (C) entlang der Längsachse (L) ändert.

15. Der Bohrer (10) nach einem der vorhergehenden Ansprüche, wobei der Schneidabschnitt (C) mindestens einen Schneideinsatz (15) umfasst.

16. Der Bohrer (10) nach Anspruch 15, wobei sich der mindestens eine Schneideinsatz (15) spiralförmig um den Bohrkern (11) mit einer ersten Steigung erstreckt.

17. Der Bohrer (10) nach Anspruch 16, wobei entlang der Längsachse (L) die äussersten Punkte (12) der Querschnitte entlang einer spiralförmigen Linie mit einer zweiten Steigung angeordnet sind, wobei die erste Steigung und die zweite Steigung sich voneinander unterscheiden, wobei die erste Steigung vorzugsweise kleiner ist als die zweite Steigung.

18. Der Bohrer (10) nach einem der vorhergehenden Ansprüche, wobei der Bohrer ferner mindestens ein Führungs-Gewinde (19) umfasst, das spiralförmig um den Bohrkern (11) ausgebildet ist und vorzugsweise als ein einzelnes Gewinde ausgebildet ist.

## Revendications

1. Un foret (10) comprenant :
une extrémité apicale (1), une extrémité coronale (2), un axe longitudinal (L) s'étendant entre l'extrémité apicale et l'extrémité coronale,
un noyau de foret (11), et
une partie coupante (C) s'étendant au moins partiellement le long du noyau de foret ;
dans lequel un contour d'une section transversale de la partie coupante perpendiculaire à l'axe longitudinal comprend au moins un point le plus extérieur (12) à une première distance radiale (r1) de l'axe longitudinal et au moins un point de coupe (14) à une seconde distance radiale (r2) de l'axe longitudinal ; et
dans lequel le long d'une première partie de la partie coupante, la seconde distance radiale est inférieure à la première distance radiale, et le long d'une seconde partie de la partie coupante, la seconde distance radiale est sensiblement égale à la première distance radiale.
et dans lequel la première partie de la partie coupante (C) est positionnée apicalement par rapport à la seconde partie de la partie coupante.

2. Foret (10) selon la revendication 1, dans lequel le foret comprend en outre une partie non coupante (N) s'étendant le long du noyau de foret (11), dans lequel le long de la partie non coupante un contour d'une section transversale du noyau de foret perpendiculaire à l'axe longitudinal (L) comprend au moins un point le plus extérieur (12) à une première distance radiale (r1) de l'axe longitudinal.

3. Foret (10) selon la revendication 2, dans lequel la partie non coupante (N) est positionnée apicalement par rapport à la partie coupante (C).

4. Foret (10) selon la revendication 1 comprenant en outre :
une partie non coupante (N) s'étendant le long du noyau de foret ;
dans lequel, le long de la partie non coupante, un contour d'une section transversale du noyau de foret perpendiculaire à l'axe longitudinal comprend au moins un point le plus extérieur (12) à une première distance radiale (r1) de l'axe longitudinal,
dans lequel la partie non coupante est positionnée apicalement par rapport à la partie coupante.

5. Foret (10) selon la revendication 4, dans lequel la partie coupante (C) comprend au moins une partie le long de l'axe longitudinal, où la deuxième distance radiale (r2) est inférieure à la première distance radiale (r1).

6. Foret (10) selon la revendication 4 ou 5, dans lequel la partie non coupante (N) du noyau de foret (11) comprend une zone de compression et une zone de relaxation.

7. Foret (10) selon l'une quelconque des revendications 4 à 6, dans lequel le contour d'une section transversale le long de la partie non coupante (N) du noyau de foret (11) et perpendiculaire à l'axe longitudinal (L) est non circulaire.

8. Foret (10) selon l'une quelconque des revendications précédentes, dans lequel la partie coupante (C) du noyau de foret (11) comprend une zone de compression (17) et une zone de relaxation (18).

9. Foret (10) selon la revendication 8, dans lequel le point de coupe (14) est positionnée à l'intérieur de la zone de compression.

10. Foret (10) selon l'une quelconque des revendications précédentes, dans lequel le contour d'une section transversale le long de la partie coupante (C) du noyau de foret (11) et perpendiculaire à l'axe longitudinal (L) est non circulaire.

11. Foret (10) selon l'une quelconque des revendications précédentes, s'étendant de l'extrémité apicale (1) vers l'extrémité coronale (2), le noyau de foret (11) comprenant en outre une partie de guidage (16), la section transversale de la partie de guidage perpendiculaire à l'axe longitudinal (L) étant de préférence circulaire.

12. Foret (10) selon l'une quelconque des revendications précédentes, dans lequel la section transversale du noyau de foret (11) a deux ou trois paires de points les plus extérieurs (12) et de pointes de coupe (14), chaque paire comprenant un point le plus extérieur et une pointe de coupe.

13. Foret (10) selon l'une quelconque des revendications précédentes, dans lequel, dans une direction de rotation prédéterminée du foret, l'emplacement de la pointe de coupe (14) change, passant d'un emplacement avant le point le plus extérieur (12) à un emplacement après le point le plus extérieur dans une direction apicale-coronale.

14. Foret (10) selon l'une quelconque des revendications précédentes, dans lequel le rapport entre la première distance radiale (r1) et la deuxième distance radiale (r2) entre les sections transversales de la pointe de coupe (C) le long de l'axe longitudinal (L) change.

15. Foret (10) selon l'une quelconque des revendications précédentes, dans lequel la partie coupante (C) comprend au moins une goujure de coupe (15).

16. Foret (10) selon la revendication 15, dans lequel au moins une goujure de coupe (15) s'étend en hélice autour du noyau de foret (11) avec un premier pas.

17. Foret (10) selon la revendication 16, dans lequel, le long de l'axe longitudinal (L), les points les plus extérieurs (12) des sections transversales sont situés le long d'une ligne hélicoïdale ayant un deuxième pas, dans lequel le premier pas et le deuxième pas diffèrent l'un de l'autre, le premier pas étant de préférence plus petit que le deuxième pas.

18. Foret (10) selon l'une quelconque des revendications précédentes, dans lequel le foret comprend en outre au moins un filet de guidage (19) formé en hélice autour du noyau de foret (11) et de préférence formé comme un seul filet.
